# EUROPEAN PATENT APPLICATION

(11) **EP 3 574 819 A1**
(43) Date of publication of application: **04.12.2019**
(21) Application number: 17894193.6
(22) Date of filing: 15.12.2017
(51) Int. Cl.: A61B 1/07, A61B 1/00, F21V 9/08, G02B 21/00, G02B 23/26

(54) **LIGHT SOURCE DEVICE, METHOD FOR ADJUSTING LIGHT QUANTITY DISTRIBUTION, AND IMAGE ACQUISITION SYSTEM**

(30) Priority: 24.01.2017 JP 2017010125
(71) Applicant: Sony Corporation, Tokyo 108-0075 (JP)
(72) Inventor: UEDA, Mitsunori, Tokyo 108-0075 (JP); OKI, Tomoyuki, Tokyo 108-0075 (JP)
(74) Representative: D Young & Co LLP
(86) International application number: PCT/JP2017/045107
(87) International publication number: WO 2018/139101

(57) **Abstract**

To homogenize radiation angles of two kinds of lights and to efficiently use the lights while restricting an increase in manufacture cost when multiplexing a white illumination light and a narrowband light.

A light source apparatus according to the present disclosure includes a white illumination light source configured to emit a white illumination light, one or more narrowband light sources configured to emit a narrowband light, a multiplexing optical system configured to multiplex the white illumination light and the narrowband light, a condensing optical system configured to couple the multiplexed white illumination light and narrowband light to a light guide provided in an image acquisition apparatus and configured to guide an illumination light into the image acquisition apparatus, and a light amount distribution adjustment device provided on an optical axis between the narrowband light source and the multiplexing optical system and configured to adjust a light amount distribution of the narrowband light, in which the light guide is a bundle-type light guide in which multimode optical fibers are bundled or a liquid light guide filling a liquid therein, and the light amount distribution adjustment device adjusts a light amount distribution of the narrowband light such that a radiation angle distribution of the narrowband light transmitting through the light amount distribution adjustment device is anisotropic.

## Description

### TECHNICAL FIELD

The present disclosure relates to a light source apparatus, a light amount distribution adjustment method, and an image acquisition system.

### BACKGROUND ART

Generally, a radiation angle of a laser light emitted from a laser light source is largely different from a radiation angle of a white light emitted from a white light source using a fluorescent substance for solid-state light emitting device. A laser light emitted from a typical visible light semiconductor laser source has a Gaussian shape with a different aspect ratio, and a white light emitted from a white light source using a fluorescent substance has a Lambertian shape. Even if the laser light and the white light are multiplexed on the same axis (even if their diameters of light fluxes are simply matched and then multiplexed by a dichroic mirror, for example), the intensity distributions of the respective lights are different, and thus the colors change depending on an irradiation position or a variation in color is caused.

In a case where such a phenomenon occurs, the colors are different within the field of view in a device for observing an object to be observed through the naked eyes especially like a microscope or the like, and thus they are likely to be a cause of fatigue of an observer and are difficult to accurately observe. Thus, an optical system such as Kohler illumination is incorporated or a fly-eye optical system is incorporated for both the lights in order to homogenize the radiation angles of the above two kinds of lights so that light fluxes with uniform intensity distributions are created and are multiplexed by a dichroic mirror or the like.

Further, in a case where an optical system as described above is not employed, the light flux with a higher intensity at the center such as Gaussian beam is simply scattered by a diffusion device (see Patent Document 1 described below, for example), a Lambertian radiation angle distribution is realized and multiplexed.

### CITATION LIST

### PATENT DOCUMENT

Patent Document 1: Japanese Patent Application Laid-Open No. 2016-120105

### SUMMARY OF THE INVENTION

### PROBLEMS TO BE SOLVED BY THE INVENTION

However, in a case where an optical system such as Kohler illumination is incorporated or a fly-eye optical system is incorporated for both lights in order to homogenize the radiation angles of the two kinds of lights, many optical devices are used, which leads to a remarkable increase in cost for realizing a light source apparatus.

Further, in a case where a diffusion device is used as disclosed in Patent Document 1 described above, as a result of an increase in light flux, a larger amount of lights are lost away from an optical axis, and lights cannot be efficiently used.

Therefore, the present disclosure proposes a light source apparatus capable of homogenizing radiation angles of two kinds of lights and efficiently using the lights while restricting an increase in manufacture cost when multiplexing a white illumination light and a narrowband light, a light amount distribution adjustment method, and an image acquisition system in terms of the above situations.

### SOLUTIONS TO PROBLEMS

According to the present disclosure, there is provided a light source apparatus including a white illumination light source configured to emit a white illumination light, one or more narrowband light sources configured to emit a narrowband light with a narrower wavelength band than the white illumination light, a multiplexing optical system configured to multiplex the white illumination light and the narrowband light, a condensing optical system configured to couple the multiplexed white illumination light and narrowband light to a light guide provided in an image acquisition apparatus and configured to guide an illumination light into the image acquisition apparatus, and a light amount distribution adjustment device provided on an optical axis between the narrowband light source and the multiplexing optical system and configured to adjust a light amount distribution of the narrowband light, in which the light guide is a bundle-type light guide in which multimode optical fibers are bundled or a liquid light guide filling a liquid therein, and the light amount distribution adjustment device adjusts a light amount distribution of the narrowband light such that a radiation angle distribution of the narrowband light transmitting through the light amount distribution adjustment device is anisotropic.

Further, according to the present disclosure, there is provided a light amount distribution adjustment method including putting a narrowband light emitted from one or more narrowband light sources configured to emit a narrowband light with a narrower wavelength band than a white illumination light emitted from a white illumination light source into a light amount distribution adjustment device configured to adjust a light amount distribution of the narrowband light, multiplexing the narrowband light with the adjusted light amount distribution and the white illumination light by a multiplexing optical system, and coupling the multiplexed white illumination light and narrowband light to a light guide by a condensing optical system configured to couple the lights to the light guide provided in an image acquisition apparatus and configured to guide an illumination light into the image acquisition apparatus, in which the light guide is a bundle-type light guide in which multimode optical fibers are bundled or a liquid light guide filling a liquid therein, and the light amount distribution adjustment device adjusts a light amount distribution of the narrowband light such that a radiation angle distribution of the narrowband light transmitting through the light amount distribution adjustment device is anisotropic.

Furthermore, according to the present disclosure, there is provided an image acquisition system including a light source apparatus configured to multiplex a white illumination light and a narrowband light with a narrower wavelength band than the white illumination light and to emit the multiplexed lights to the outside, and an image acquisition apparatus configured to acquire an image of an object to be observed by use of a light emitted from the light source apparatus, in which the image acquisition apparatus includes a light guide configured to guide an illumination light into the image acquisition apparatus, the light guide is a bundle-type light guide in which multimode optical fibers are bundled or a liquid light guide filling a liquid therein, the light source apparatus includes a white illumination light source configured to emit the white illumination light, one or more narrowband light sources configured to emit the narrowband light, a multiplexing optical system configured to multiplex the white illumination light and the narrowband light, a condensing optical system configured to couple the multiplexed white illumination light and narrowband light to the light guide provided in the image acquisition apparatus, and a light amount distribution adjustment device provided on an optical axis between the narrowband light source and the multiplexing optical system and configured to adjust a light amount distribution of the narrowband light, and the light amount distribution adjustment device adjusts a light amount distribution of the narrowband light such that a radiation angle distribution of the narrowband light transmitting through the light amount distribution adjustment device is anisotropic.

According to the present disclosure, a narrowband light emitted from the narrowband light source is adjusted in its light amount distribution by the light amount distribution adjustment device such that a radiation angle distribution is anisotropic, and then multiplexed with a white illumination light emitted from the white illumination light source by the multiplexing optical system. The narrowband light with the adjusted light amount distribution, and the white illumination light are coupled to a predetermined light guide provided in the image acquisition apparatus by the condensing optical system.

### EFFECTS OF THE INVENTION

As described above, according to the present disclosure, it is possible to homogenize radiation angles of two kinds of lights and to efficiently use the lights while restricting an increase in manufacture cost when multiplexing a white illumination light and a narrowband light.

Additionally, the above effect is not necessarily restrictive, and any effect described in the present specification or other effect graspable from the present specification may be obtained together with the above effect or instead of the above effect.

### BRIEF DESCRIPTION OF DRAWINGS

Fig. 1 is an explanatory diagram illustrating a relationship between a length of a light guide and an intensity distribution of a light radiated from an exit end of the light guide.
Fig. 2A is a graph illustrating exemplary radiation angle patterns of a white illumination light and a narrowband light.
Fig. 2B is an explanatory diagram for explaining exemplary radiation angle patterns of a white illumination light and a narrowband light.
Fig. 3 is a graph illustrating an exemplary method for adjusting a Gaussian light to a Lambertian light.
Fig. 4 is a graph illustrating exemplary radiation angle patterns of a white illumination light, a narrowband light, and an enlarged narrowband light.
Fig. 5 is a graph illustrating an exemplary relationship between an enlarging magnification and a radiation angle pattern of a narrowband light.
Fig. 6 is a block diagram schematically illustrating an exemplary entire configuration of an image acquisition system according to an embodiment of the present disclosure.
Fig. 7 is an explanatory diagram schematically illustrating an exemplary narrowband light source according to the embodiment.
Fig. 8A is an explanatory diagram schematically illustrating an exemplary light amount distribution adjustment device according to the embodiment.
Fig. 8B is an explanatory diagram schematically illustrating exemplary light amount distribution adjustment devices according to the embodiment.
Fig. 8C is an explanatory diagram schematically illustrating an exemplary configuration of a light source apparatus according to the embodiment.
Fig. 9A is an explanatory diagram schematically illustrating other exemplary light amount distribution adjustment device according to the embodiment.
Fig. 9B is an explanatory diagram schematically illustrating other exemplary configuration of the light source apparatus according to the embodiment.
Fig. 10 is an explanatory diagram schematically illustrating other exemplary configuration of the light source apparatus according to the embodiment.
Fig. 11 is an explanatory diagram for explaining radiation patterns of a narrowband light.
Fig. 12 is an explanatory diagram for explaining light amount distribution adjustment of a narrowband light according to the embodiment.
Fig. 13 is an explanatory diagram for explaining how a narrowband light is incident into a condensing optical system.
Fig. 14 is a graph illustrating exemplary intensity distributions of an isotropic narrowband light at the exit end of the light guide.
Fig. 15 is an explanatory diagram for explaining how a narrowband light is incident into the condensing optical system according to the embodiment.
Fig. 16 is a graph illustrating exemplary intensity distributions of a narrowband light at the exit end of the light guide.
Fig. 17A is an explanatory diagram illustrating an exemplary intensity distribution of a narrowband light in a case where an isotropic diffusion plate is used.
Fig. 17B is a graph illustrating an exemplary intensity distribution of a narrowband light in a case where the isotropic diffusion plate is used.
Fig. 18A is an explanatory diagram illustrating an exemplary intensity distribution of a narrowband light in a case where the isotropic diffusion plate is used.
Fig. 18B is a graph illustrating an exemplary intensity distribution of a narrowband light in a case where the isotropic diffusion plate is used.
Fig. 19A is an explanatory diagram illustrating an exemplary intensity distribution of a narrowband light in a case where the isotropic diffusion plate is used.
Fig. 19B is a graph illustrating an exemplary intensity distribution of a narrowband light in a case where the isotropic diffusion plate is used.
Fig. 20A is an explanatory diagram illustrating an exemplary intensity distribution of a narrowband light in a case where the isotropic diffusion plate is used.
Fig. 20B is a graph illustrating an exemplary intensity distribution of a narrowband light in a case where the isotropic diffusion plate is used.
Fig. 21A is an explanatory diagram illustrating an exemplary intensity distribution of a narrowband light in a case where an anisotropic diffusion plate is used.
Fig. 21B is a graph illustrating an exemplary intensity distribution of a narrowband light in a case where the anisotropic diffusion plate is used.
Fig. 22A is an explanatory diagram illustrating an exemplary intensity distribution of a narrowband light in a case where the anisotropic diffusion plate is used.
Fig. 22B is a graph illustrating an exemplary intensity distribution of a narrowband light in a case where the anisotropic diffusion plate is used.
Fig. 23A is an explanatory diagram illustrating an exemplary intensity distribution of a narrowband light in a case where the anisotropic diffusion plate is used.
Fig. 23B is a graph illustrating an exemplary intensity distribution of a narrowband light in a case where the anisotropic diffusion plate is used.
Fig. 24A is an explanatory diagram illustrating an exemplary intensity distribution of a narrowband light in a case where the anisotropic diffusion plate is used.
Fig. 24B is a graph illustrating an exemplary intensity distribution of a narrowband light in a case where the anisotropic diffusion plate is used.
Fig. 25A is an explanatory diagram illustrating an exemplary intensity distribution of a narrowband light in a case where the anisotropic diffusion plate is used.
Fig. 25B is a graph illustrating an exemplary intensity distribution of a narrowband light in a case where the anisotropic diffusion plate is used.
Fig. 26A is an explanatory diagram schematically illustrating an exemplary multiplexing optical system in the light source apparatus according to the embodiment.
Fig. 26B is an explanatory diagram schematically illustrating other exemplary multiplexing optical system in the light source apparatus according to the embodiment.

### MODE FOR CARRYING OUT THE INVENTION

A preferred embodiment of the present disclosure will be described below in detail with reference to the accompanying drawings. Additionally, constituents having substantially the same functional configurations are denoted with the same reference numerals, respectively, and a repeated description thereof will be omitted in the present specification and the drawings.

Additionally, the description will be made in the following order.
1. Examination contents by present inventors and their results
2. Intensity distribution adjustment method according to present disclosure
3. Embodiment
   3-1. Entire configuration of image acquisition system
   3-2. Configuration of light source apparatus
4. Conclusion

### (Examination Contents by present inventors and their results)

Examination contents by the present inventors and their results for the above problems will be described in detail prior to describing a light source apparatus, a light amount distribution adjustment method, and an image acquisition system according to an embodiment of the present disclosure.

The light source apparatus according to the embodiment of the present disclosure described below in detail is applicable as a light source for various endoscopes, microscopes, and the like used in various surgeries and the like in the medical institutions. Here, a white light source (such as halogen lamp or xenon lamp, for example) similar to sunlight or a fluorescent light source (such as light source using blue-light excited yellow fluorescent substance or ultraviolet-ray excited white fluorescent substance) is used as a light source in typical endoscopes or microscopes including industrial ones.

However, a medical endoscope or microscope may further include a special-light light source in addition to the above-described ones for white light in order to make specific observations. The observation methods using such a special-light light source include an observation method called narrow band imaging (NBI) in which a light is emitted with part of its wavelength band emphasized thereby to make a change in disease or body tissue which is difficult to view easily viewable, an observation method for observing a blood flow or the like in the blood vessels by combining or administering fluorescent substances sensitive to visible ray or infrared ray, specifying a position by causing a lesion to emit a light by fluorescent substances specifically collected on a tumor site, or determining a stepwise change in state by the magnitude of an emitted light's intensity, and the like, for example. During an observation using such a special light, the illumination range of the special light is preferably the same as the illumination range of the white light to the extent possible, and the intensity distribution of the special light is preferably similar to the intensity distribution of the white light.

Further, in a case where the fluorescent substance emits a light in the invisible region such as infrared wavelength band, image observation is made by use of an imaging device sensitive to fluorescently-emitted light so that the intensity distribution of the special light can be corrected from a shot image. However, in a case where an observation is made by the naked eyes or in consideration of various imaging devices, it is preferable that the light irradiation intensity distributions for both a white light and a special light are such that the irradiation intensity is high around the center of an irradiation region, changes as little as possible in a wide range of the irradiation region, and the irradiation intensity continuously decreases toward the periphery of the irradiation region like the intensity distributions obtained by typical lamp illuminations in order to make natural observations.

In this way, an endoscope or microscope used in medical practices such as surgeries includes a special light in addition to an illumination light source configured of typical white light, and in this case, it is important that the irradiation intensity and the size of the irradiation region of the special light need to be adjusted to those of the white light.

A white illumination light emitted from a light source using typical fluorescent substance or the like has a Lambertian-approximated radiation distribution, and thus a large amount of lights are not radiated ahead and an optical system with excellent use efficiency of light is difficult to configure. On the other hand, various lasers such as laser diode are lights (narrowband lights) with a narrow wavelength band which are frequently used as special-light light source as described above, and emit a light in a spotlight-shaped radiation distribution in which the intensity at the center of the irradiation region is so high. A Lambertian light can be used as an illumination light with a wide irradiation region as it is, and it is preferable that the light is propagated while the radiation angle distribution is kept to the extent possible. Thus, when the irradiation intensity and the size of the irradiation region of the white light are adjusted to the irradiation intensity and the size of the irradiation region of the special light, it is preferable that the irradiation intensity and the size of the irradiation region of the special light are made close to the irradiation intensity and the size of the irradiation region of the white light.

Some methods can be employed in order to make a spotlight-shaped laser light close to a Lambertian light. For example, there can be assumed two simple methods of (1) a laser light is incident into an optical device (or diffusion plate) for diffusing lights such as frosted glass or sandblasted glass and a diffused light emitted from the optical device (diffusion plate) is used as a light source and (2) a laser light is enlarged by use of an optical system for enlarging only the center of a laser light and only the part having the uniform amount of light in the high-intensity center is used as a light source. However, both the methods are low in use efficiency of light energy since it is basically important to design an optical system to eliminate so strong lights. That is, more power for driving a laser light is required in order to obtain the same amount of light as the white light.

Incidentally, when a narrowband light is incident into one end of one light guide, the incident narrowband light repeatedly reflects on the side of the light guide to be guided to the other end of the light guide. At this time, there is known that the narrowband light incident into the light guide is averaged in the light guide to be a concentric exit light. That is, the incident narrowband light is converted into a concentric light in the light guide, and is radiated from the other end of the light guide while the angle (incident angle) of the light incident into the light guide is kept.

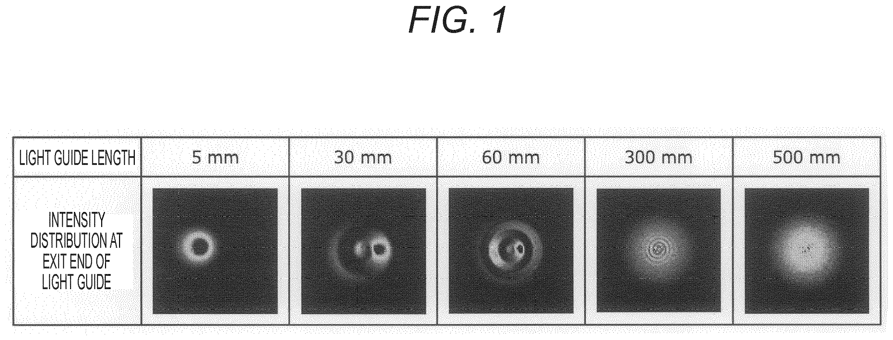

Fig. 1 illustrates simulation results as to whether an incident laser light is converted into a concentric light how long the light guide is (light guide length), the light guide including an optical fiber having a core with a diameter of 4.5 mm (the core diameter is of a typical light guide). As is clear from Fig. 1, it can be seen that in a case where the light guide length is 5 mm, 30 mm, and 60 mm, the intensity distribution of a light radiated from the exit end of the light guide is not sufficiently averaged. Further, it can be seen from Fig. 1 that when the light guide length is 300 mm, the intensity distribution of a light radiated from the exit end of the light guide is closer to a concentric circle but a spiral intensity distribution slightly remains. On the other hand, it can be seen that when the light guide length is 500 mm, the incident laser light is almost completely converted into a concentric light. Here, a light guide mounted on a typical endoscope or microscope has a length of 500 mm or more in many case, and it is assumed that a laser light is incident into a typical light guide thereby to be able to convert the laser light into a concentric light.

Now in Japanese Patent Application Laid-Open No. 2016-137309, a plurality of narrowband lights having substantially the same wavelengths is incident into a light guide at different angles, the intensities of the respective narrowband lights having substantially the same wavelengths incident into the light guide are adjusted to be concentric and to be the same as the intensity distribution at which an intensity distribution as an envelope aims, and further a diffusion plate is arranged behind the output of the light guide so that a light with a ring-shaped intensity distribution is converted into a Lambertian light, thereby achieving uniform luminance. Further, Japanese Patent Application Laid-Open No. 2016-137309 described above describes that the focal length of a laser collimator lens is increased thereby to increase the width of a ring-shaped light and the ring-shaped light is connected to the light guide thereby to adjust the luminance distribution.

However, as a result of an examination by the present inventors, the intensity of an optical axis increases and an intensity distribution turns wavelike as long as the focal length is actually increased and the luminance distribution within an effective diameter is rarely present. That is, the optical system disclosed in Japanese Patent Application Laid-Open No. 2016-137309 described above requires a plurality of light sources for emitting narrowband lights having substantially the same wavelengths, and employs a plurality of light emitting devices for emitting narrowband lights having substantially the same wavelengths only when a failure such as a shortage of luminance is caused in a single light emitting device since it costs so much to add the light emitting devices or to add a drive circuit, for example. Further, an optical design based on a plurality of light sources is low in reliability and is difficult to employ in a situation in which desired light emitting luminance can be realized by a single light emitting device.

The present inventors have obtained the following finding on the basis of the above examination.

That is, a light source apparatus for a plurality of wavelengths having the substantially uniform luminance distributions needs to be prepared on the incident side until a light is incident into the light guide, or an intensity distribution of a light with each wavelength in the diameter direction of the light guide needs to be optimized on the incident side until a light is incident into the light guide in order to prepare a light source actually used in a hospital or the like for obtaining a uniform intensity distribution for various light guides manufactured by various manufacturers.

### (Intensity distribution adjustment method according to present disclosure)

The present inventors have made further examinations of the intensity distribution adjustment method on the basis of the above finding. The examination contents will be described below in detail.

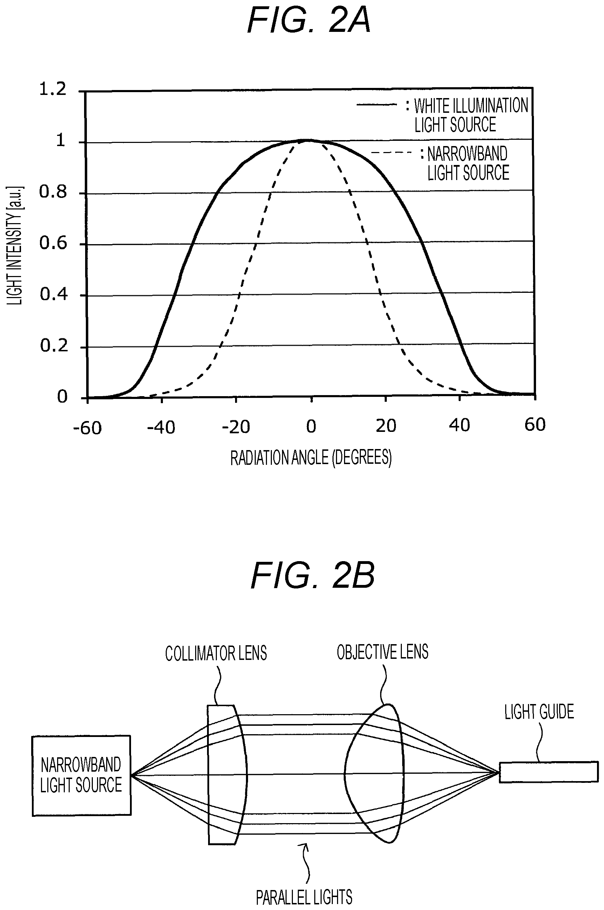

Now since a radiation angle of a narrowband light source such as various lasers is largely different from a radiation angle of a light source such as light emitting diode (LED) used as a white illumination light source, when the narrowband light and the white light are coupled to typical external light guides, respectively, and the intensity distributions (light amount distributions) at the exit end of the light guide are measured, the radiation patterns as illustrated in Fig. 2A are obtained, for example. In Fig. 2A, the vertical axis indicates an intensity at the exit end of the light guide and the horizontal axis indicates a radiation angle with respect to the optical axis position (0 degrees). As is clear from Fig. 2A, the radiation angle of the white illumination light source is around ±60 degrees while the radiation angle of the narrowband light source is around ±40 degrees.

Additionally, the above measurement result is obtained by designing parallel lights with the incident diameter of the lens, and the light flux diameter of the narrowband light and the light flux diameter of the white illumination light source almost matched by use of an objective lens with NA of around 0.6, and making simulations. Further, as schematically illustrated in Fig. 2B, the narrowband light is designed to be a parallel light by a collimator lens and then to be condensed by the objective lens, and thus the lateral magnification can be defined by the focal length of the collimator lens and the focal length of the objective lens. The lateral magnification is initially described at 1 for simplicity.

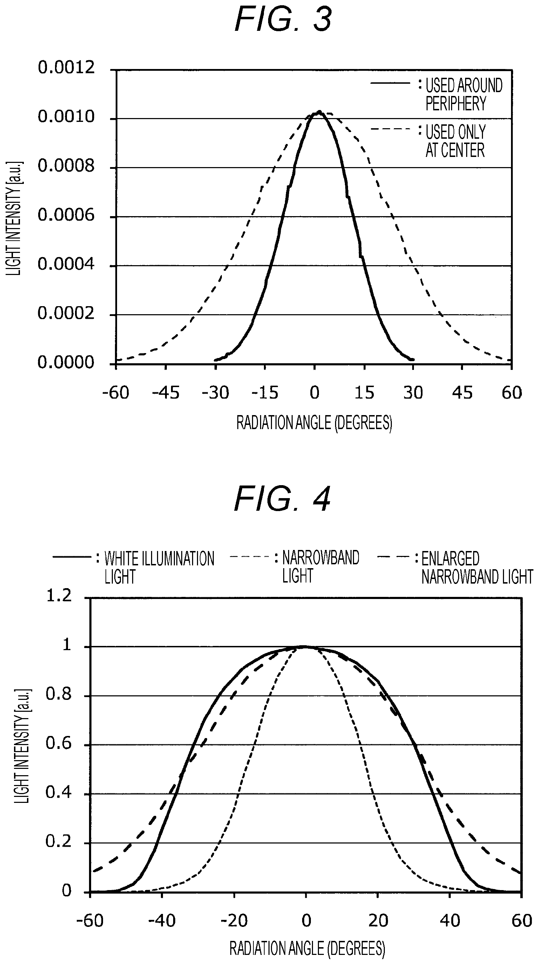

A laser light as a kind of narrowband light is high in its directivity and is easy to control, and for example, there is known that when the magnification of the laser light is changed by a predetermined well-known optical system, the radiation angle of the laser changes. Fig. 3 illustrates how the radiation angle changes in a case where the lateral magnification of the laser light is halved by a predetermined well-known optical system. As is clear fromFig. 3, the lateral magnification of the laser light is halved and only the center of the enlarged laser light is used thereby to double the radiation angle, but the light amount around the periphery of the laser light cannot be used and thus the total light amount reduces.

When such a laser light is coupled to a typical external light guide and an intensity distribution at the exit end of the light guide is measured, the results illustrated in Fig. 4 are obtained. Fig. 4 illustrates a radiation pattern of the white illumination light and a radiation pattern of the narrowband light before the change in magnification in addition to a radiation pattern of the narrowband light with an enlarged radiation angle. As is clear from Fig. 4, it can be seen that the radiation pattern of the white illumination light by LED is almost similar to the radiation pattern of the enlarged narrowband light at the center with a radiation angle of around 0 degrees, but the shapes of the radiation patterns are largely different from each other at the periphery (or at a large radiation angle). Consequently, in a case where the white illumination light and the narrowband light are multiplexed and then emitted, the luminance is different between the center and the periphery of the irradiation region.

In order to improve such a situation, as a result of further examinations by the present inventors, there has been obtained a finding that the optical axis of the narrowband light has only to be changed by use of an optical device with a higher degree of freedom in order to adjust the radiation pattern of the narrowband light to the radiation pattern of the white illumination light.

Specifically, there has been obtained a finding that a light amount distribution adjustment device capable of adjusting a light amount distribution of a narrowband light is used to adjust a light amount distribution of a narrowband light such that a radiation angle distribution of the narrowband light transmitting through the light amount distribution adjustment device is anisotropic according to the embodiment of the present disclosure described below in detail.

According to the embodiment of the present disclosure described below in detail, the light amount distribution adjustment device uses at least any of (a) a group of lenses including at least two cylindrical lenses, (b) an anisotropic diffusion plate with an anisotropic diffusion property, and (c) a multi-lens array in a cylindrical lens shape, for example.

Here, a direction in which a narrowband light travels (optical axis direction) is assumed as z-axis direction, and two directions in a plane which are orthogonal to each other with respect to the optical axis direction are assumed as x-axis direction and y-axis direction, respectively, for convenience. At this time, the enlarging magnification of a narrowband light is independently adjusted in the x-axis direction and in the y-axis direction by use of two cylindrical lenses with different focal lengths thereby to independently set a light flux in the x-axis direction and a light flux in the y-axis direction at desired radiation angles, respectively. Additionally, a light beam having the anisotropic radiation pattern is incident into various well-known light guides (more specifically, light guides with a length of 500 mm or more) . An intensity distribution in the rotation direction (direction orthogonal to the long-axis direction of the light guide) is mixed to be almost uniform, but the components of the radiation angle changed in the x-axis direction and in the y-axis direction, respectively, are kept also in the light guide. A radiation angle distribution of a narrowband light transmitting through the light guide can be adjusted in a desired distribution by use of the property of the light guide .

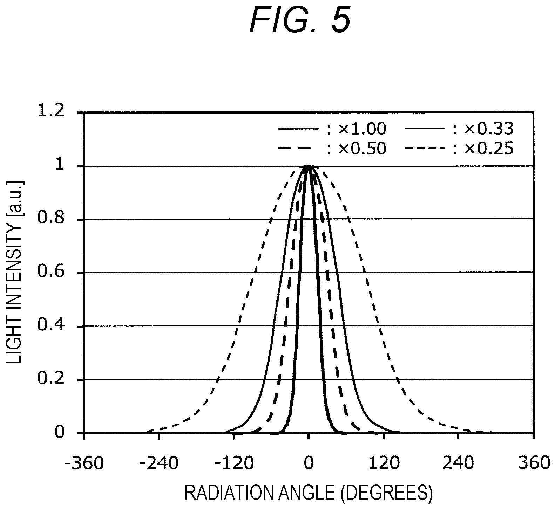

For example, as illustrated in Fig. 5, the enlarging magnification is the reciprocal of the radiation angle, where the radiation angle is a' times at the enlarging magnification of 1/a times. Thus, the light amount distribution adjustment can be made such that the enlarging magnification in the x-axis direction and the enlarging magnification in the y-axis direction of the narrowband light are appropriately selected (for example, the enlarging magnification of the narrowband light in the x-axis direction is set at 0.25 times and the radiation angle in the x-axis direction is set at nearly four times), and a variation in luminance at the center of the irradiation region is adjusted for a white illumination light (LED light) and the luminance at the periphery is set by use of the intensity distribution of the foot of a Gaussian beam in the y-axis direction.

Further, the above light amount distribution adjustment can be realized by use of an anisotropic diffusion plate with an anisotropic diffusion property. The light irradiation directions in the x-axis direction and in the y-axis direction can be independently controlled by the anisotropic diffusion plate, thereby achieving a further reduction in space and a further reduction in cost than in a case where two cylindrical lenses are used.

Further, the light amount distribution adjustment can be made by use of a multi-lens array in a cylindrical lens shape.

A light source apparatus according to the embodiment of the present disclosure based on the above findings will be described below in detail.

### (Embodiment)

### <Entire configuration of image acquisition system>

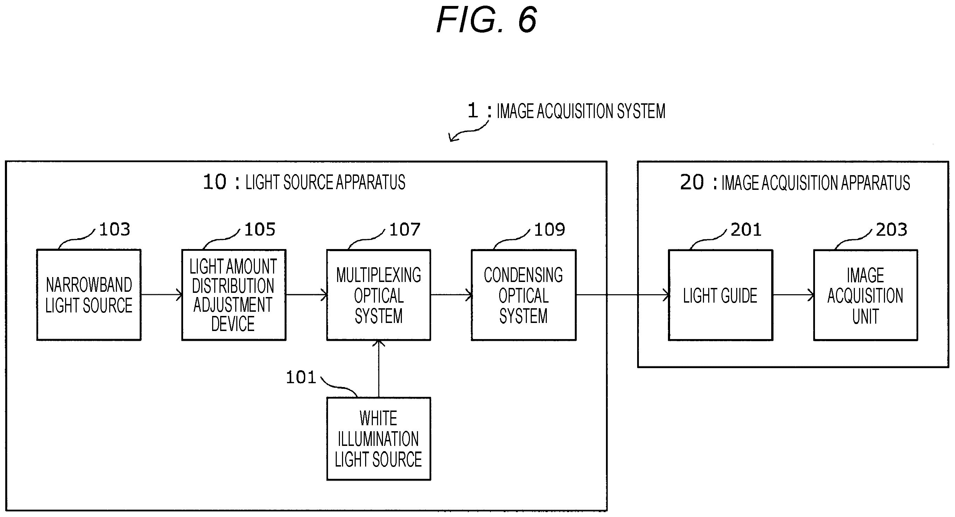

An entire configuration of an image acquisition system including a light source apparatus according to the embodiment of the present disclosure will be first described briefly with reference to Fig. 6. Fig. 6 is a block diagram schematically illustrating an exemplary entire configuration of the image acquisition system according to the present embodiment.

An image acquisition system 1 according to the present embodiment mainly includes a light source apparatus 10 and an image acquisition apparatus 20 as illustrated in Fig. 6.

The light source apparatus 10 is directed for multiplexing a white illumination light and a narrowband light with a narrower wavelength band than the white illumination light and emitting the multiplexed lights to the outside. A configuration of the light source apparatus 10 will be described below in detail.

The image acquisition apparatus 20 is directed for acquiring an image of an object to be observed by use of a light emitted from the light source apparatus 10. The image acquisition apparatus 20 mainly includes a light guide 201 for guiding an illumination light into the image acquisition apparatus 20, and an image acquisition unit 203 for acquiring an image of an object to be observed as illustrated in Fig. 6.

In the image acquisition apparatus 20 according to the present embodiment, the light guide 201 is a bundle-type light guide in which multimode optical fibers are bundled or a liquid light guide filling a liquid therein. The light guide 201 preferably has a length of 500 mm or more. A light beam incident into the light guide 201 is guided to the image acquisition unit 203 at a later stage, and is used as an illumination light for acquiring an image of an object to be observed.

The image acquisition unit 203 is directed for acquiring an image of an object to be observed by use of the illumination light guided by the light guide 201. The image acquisition unit 203 has various optical systems for guiding an illumination light and an image of an object to be observed, and various imaging devices for shooting an image of an object to be observed guided by the optical systems. The image acquisition unit 203 can employ an endoscope unit for various industrial or medical endoscope apparatuses, a microscope unit for various industrial or medical microscope apparatuses, or the like.

The light source apparatus 10 and the image acquisition apparatus 20 function in association with each other, and thus the image acquisition system 1 according to the present embodiment can acquire an image of an object to be observed.

The image acquisition system 1 according to the present embodiment has been briefly described above with reference to Fig. 6.

### <Configuration of light source apparatus>

### [Entire configuration]

The light source apparatus 10 according to the present embodiment will be subsequently described in detail with reference to Fig. 6 to Fig. 26B.

The light source apparatus 10 is directed for multiplexing a white illumination light and a narrowband light with a narrower wavelength band than the white illumination light and emitting the multiplexed lights to the outside as described earlier. The light source apparatus 10 mainly includes a white illumination light source 101, a narrowband light source 103, a light amount distribution adjustment device 105, a multiplexing optical system 107, and a condensing optical system 109, for example, as illustrated in Fig. 6.

The white illumination light source 101 is directed for emitting a white illumination light. The white illumination light source 101 is not particularly limited, and can use a well-known light source. The white illumination light source 101 may be a fluorescent light source for exciting a well-known fluorescent substance such as blue-light excited yellow fluorescent substance or ultraviolet-ray excited white fluorescent substance by various laser lights or LED lights, or a white light source such as halogen lamp or xenon lamp, for example. A white illumination light emitted from the white illumination light source 101 is a Lambertian light.

The narrowband light source 103 is directed for emitting a narrowband light with a narrower wavelength band than the white illumination light. In the light source apparatus 10 according to the present embodiment, the number of narrowband light sources 103 is not particularly limited, and one narrowband light source 103 or a plurality of narrowband light sources 103 may be employed. Narrowband lights emitted from the narrowband light source 103 may be various visible lights belonging to the visible-light wavelength band such as red light, green light, blue light, and violet light, or may be an infrared ray belonging to the infrared wavelength band, or the lights may be mutually multiplexed.

Any narrowband light source 103 according to the present embodiment capable of emitting at least any of the above narrowband lights can be used, and a plurality of light sources can be combined for use.

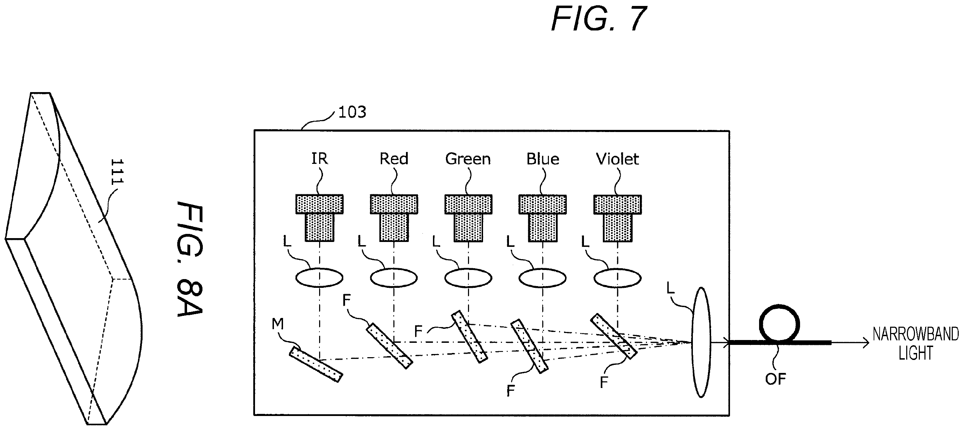

Fig. 7 schematically illustrates one light source usable for the narrowband light source 103 according to the present embodiment. The narrowband light source 103 according to the present embodiment preferably has a plurality of solid-state light sources as illustrated in Fig. 7, for example. A Gaussian light with a predetermined wavelength is emitted from each solid-state light source. Here, a combination of wavelengths of lights emitted from each solid-state light source is not particularly limited, but solid-state light sources capable of emitting an infrared ray IR, a red light Red, a green light Green, a blue light Blue, and a violet light Violet may be combined as illustrated in Fig. 7, for example. The solid-state light sources are combined as needed and operated, thereby emitting a narrowband light in a desired wavelength band at any timing.

A light emitted from each solid-state light source is controlled in its propagation direction by a lens L, a mirror M, and an optical filter F provided behind each solid-state light source, and is finally mixed by the lens L provided behind the mirror M and the optical filter F. Here, the mirror M has an optical property to reflect a light emitted from the solid-state light source, and each optical filter F has an optical property to reflect a light from the solid-state light source provided upstream of each optical filter F and to transmit lights in other wavelength bands. The mixed light is guided by an optical fiber OF, and a light emitted from the end of the optical fiber OF is used as a narrowband light.

The light amount distribution adjustment device 105 is provided on an optical axis between the narrowband light source 103 and the multiplexing optical system 107 described below, and adjusts a light amount distribution of a narrowband light emitted from the narrowband light source 103. Specifically, the light amount distribution adjustment device 105 adjusts a light amount distribution of a narrowband light such that a radiation angle distribution of the narrowband light transmitting through the light amount distribution adjustment device 105 is anisotropic as described earlier.

In the light source apparatus 10 according to the present embodiment, the light amount distribution adjustment device 105 preferably uses at least any of (a) a group of lenses including at least two cylindrical lenses, (b) an anisotropic diffusion plate having an anisotropic diffusion property, and (c) a multi-lens array in a cylindrical lens shape as described earlier. At least any of the optical devices (a) to (c) is used, and in the case of (a) or (c) described above, the focal lengths of the cylindrical lenses are appropriately selected, and in the case of (b) described above, a diffusion angle of the anisotropic diffusion plate is appropriately selected, thereby more accurately enabling a radiation angle distribution of a narrowband light transmitting through the light amount distribution adjustment device 105 to be anisotropic.

The multiplexing optical system 107 is directed for multiplexing a white illumination light emitted from the white illumination light source 101 and a narrowband light transmitting through the light amount distribution adjustment device 105 (or a narrowband light with an anisotropic radiation angle distribution) . The multiplexing optical system 107 can be configured by use of one or more well-known optical devices, and preferably includes a well-known optical device such as dichroic mirror, for example.

The condensing optical system 109 for coupling the multiplexed white illumination light and narrowband light to the light guide 201 provided in the image acquisition apparatus 20 is provided behind the multiplexing optical system 107. The multiplexed white illumination light and narrowband light are coupled to the light guide 201 by the condensing optical system 109 so that the multiplexed white illumination light and narrowband light are appropriately homogenized in the light guide 201 and a radiation angle distribution of the white illumination light and a radiation angle distribution of the narrowband light are adjusted at the exit end of the light guide 201. The condensing optical system 109 can be configured by use of one or more well-known optical devices such as various lenses.

The entire configuration of the light source apparatus 10 according to the present embodiment has been described above with reference to Fig. 6.

Additionally, in a case where a dichroic mirror is used for the multiplexing optical system 107 in the light source apparatus 10 according to the present embodiment described above, it is preferable that the angle of a light incident into the dichroic mirror is paid attention to. That is, a predetermined set incident angle is provided for the optical device such as dichroic mirror in order to realize desired transmission property and reflection property. In a case where the incident angle of a light into the optical device is not restricted within a range of around ±5 degrees of the set incident angle (more preferably within a range of around ±3 degrees of the set incident angle), the transmission property and the reflection property of the optical device such as dichroic mirror can be remarkably deteriorated.

Thus, it is preferable that collimator optical systems for turning a narrowband light and a white illumination light into collimated lights are provided on an optical axis between the light amount distribution adjustment device 105 and the multiplexing optical system 107 and on an optical axis between the white illumination light source 101 and the multiplexing optical system 107, respectively, in the light source apparatus 10 according to the present embodiment. A narrowband light and a white illumination light incident into the multiplexing optical system 107 are to be almost collimated lights by the collimator optical systems, respectively, so that the narrowband light and the white illumination light more efficiently pass inside the multiplexing optical system 107.

The configuration of the light source apparatus 10 according to the present embodiment will be more specifically described below with reference to Fig. 8A to Fig. 10.

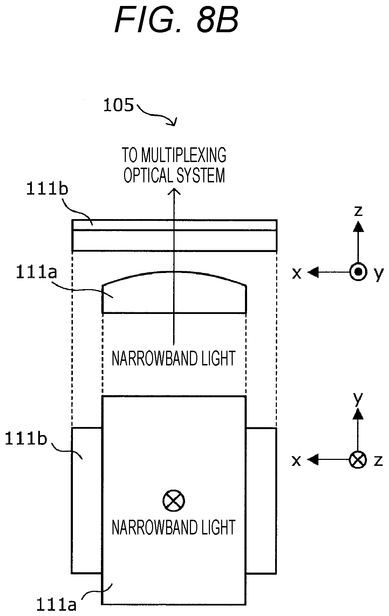

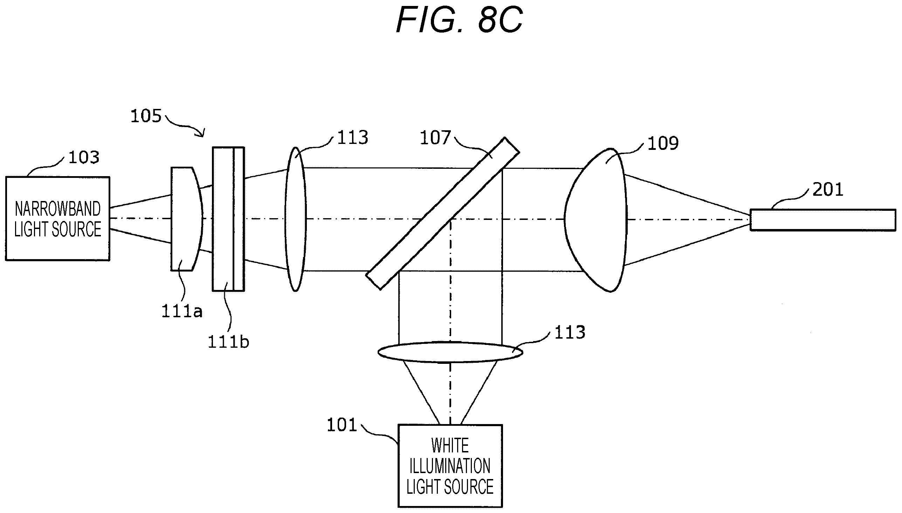

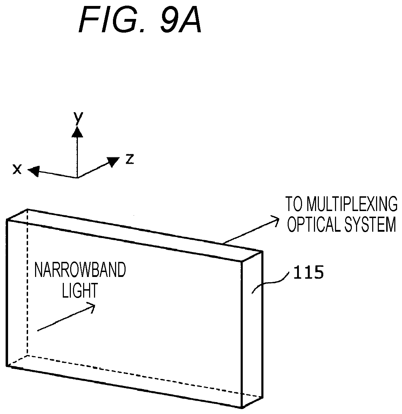

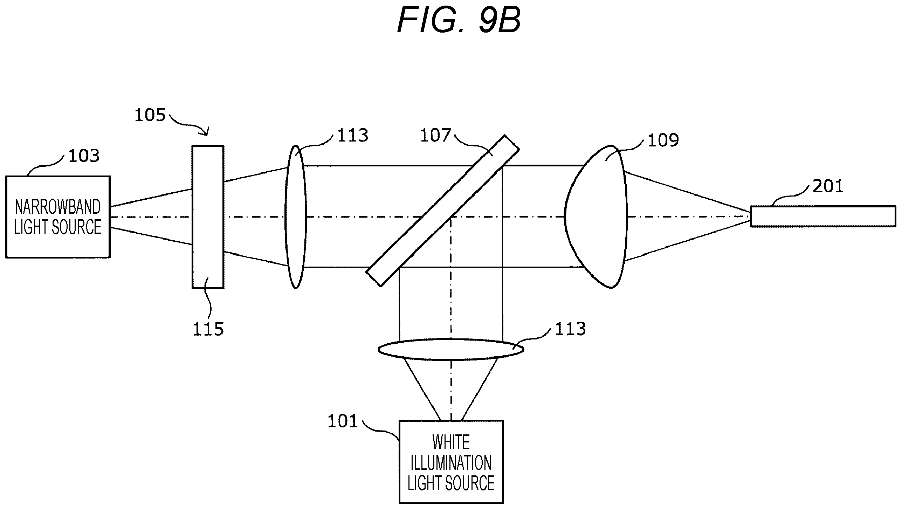

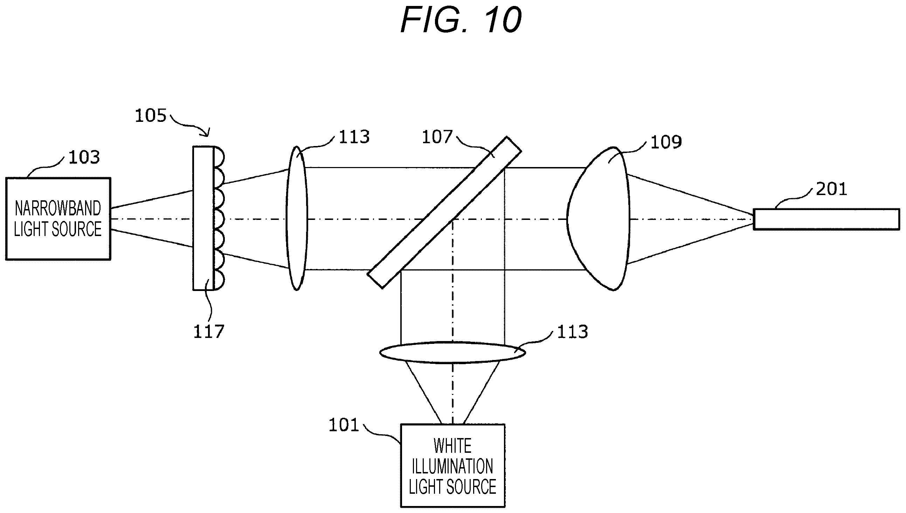

Fig. 8A and Fig. 8B are explanatory diagrams schematically illustrating an exemplary light amount distribution adjustment device according to the present embodiment, and Fig. 8C is an explanatory diagram schematically illustrating an exemplary configuration of the light source apparatus according to the present embodiment. Fig. 9A is an explanatory diagram schematically illustrating other exemplary light amount distribution adjustment device according to the embodiment, and Fig. 9B is an explanatory diagram schematically illustrating other exemplary configuration of the light source apparatus according to the present embodiment. Fig. 10 is an explanatory diagram schematically illustrating other exemplary configuration of the light source apparatus according to the present embodiment.

### [Specific example 1]

In the light source apparatus 10 according to the present embodiment, a cylindrical lens 111 as illustrated in Fig. 8A can be used for the light amount distribution adjustment device 105. At this time, as schematically illustrated in Fig. 8B, two cylindrical lenses 111a and 111b with different focal lengths are used and the cylindrical lenses 111a and 111b are combined in a cross shape thereby to independently adjust the enlarging magnification of a narrowband light in the x-axis direction and the enlarging magnification of the narrowband light in the y-axis direction in Fig. 8B, in the light source apparatus 10 according to the present embodiment.

Fig. 8C schematically illustrates optical paths in a case where the two cylindrical lenses 111a and 111b are used for the light amount distribution adjustment device 105.

In the case of the example illustrated in Fig. 8C, a white illumination light emitted from the white illumination light source 101 transmits through the collimator optical system 113 to be a collimated light, which is guided to the multiplexing optical system 107. On the other hand, a narrowband light emitted from the narrowband light source 103 transmits through the cylindrical lenses 111a and 111b functioning as the light amount distribution adjustment device 105 to have an anisotropic radiation angle distribution. Thereafter, the narrowband light transmitting through the cylindrical lenses 111a and 111b transmits through the collimator optical system 113 to be a collimated light, which is guided to the multiplexing optical system 107.

The dichroic mirror included in the multiplexing optical system 107 has an optical property to reflect a white illumination light and to transmit a narrowband light. The white illumination light and the narrowband light are mutually multiplexed via the multiplexing optical system 107 to be guided to the condensing optical system 109. The multiplexed white illumination light and narrowband light are coupled to one end of the light guide 201 of the image acquisition apparatus 20 by the condensing optical system 109.

Additionally, an isotropic diffusion plate can be arranged on the optical axis in front of and behind the group of cylindrical lenses in the optical system as illustrated in Fig. 8C.

### [Specific example 2]

In the light source apparatus 10 according to the present embodiment, an anisotropic diffusion plate 115 as illustrated in Fig. 9A can be used for the light amount distribution adjustment device 105. The anisotropic diffusion plate 115 is different in terms of the radiation property in the x-axis direction and the radiation property in the y-axis direction illustrated in Fig. 9A, and can independently adjust the radiation angles of a narrowband light in the x-axis direction and in the y-axis direction, respectively.

Fig. 9B schematically illustrates optical paths in a case where the anisotropic diffusion plate 115 is used for the light amount distribution adjustment device 105.

In the case of the example illustrated in Fig. 9B, a white illumination light emitted from the white illumination light source 101 transmits through the collimator optical system 113 to be a collimated light, which is guided to the multiplexing optical system 107. On the other hand, a narrowband light emitted from the narrowband light source 103 transmits through the anisotropic diffusion plate 115 functioning as the light amount distribution adjustment device 105 to have an anisotropic radiation angle distribution. Thereafter, the narrowband light transmitting through the anisotropic diffusion plate 115 transmits through the collimator optical system 113 to be a collimated light, which is guided to the multiplexing optical system 107.

The dichroic mirror included in the multiplexing optical system 107 has an optical property to reflect a white illumination light and to transmit a narrowband light. The white illumination light and the narrowband light are mutually multiplexed via the multiplexing optical system 107 to be guided to the condensing optical system 109. The multiplexed white illumination light and narrowband light are coupled to one end of the light guide 201 of the image acquisition apparatus 20 by the condensing optical system 109.

### [Specific example 3]

In the light source apparatus 10 according to the present embodiment, a multi-lens array in a cylindrical lens shape can be used for the light amount distribution adjustment device 105. Each single lens configuring the multi-lens array is configured in a cylindrical lens shape so that the effects similar to those of the cylindrical lenses 111a and 111b or the anisotropic diffusion plate 115 can be realized.

Fig. 10 schematically illustrates optical paths in a case where the multi-lens array 117 is used for the light amount distribution adjustment device 105.

In the case of the example illustrated in Fig. 10, a white illumination light emitted from the white illumination light source 101 transmits through the collimator optical system 113 to be a collimated light, which is guided to the multiplexing optical system 107. On the other hand, a narrowband light emitted from the narrowband light source 103 transmits through the multi-lens array 117 functioning as the light amount distribution adjustment device 105 to have an anisotropic radiation angle distribution. Thereafter, the narrowband light transmitting through the multi-lens array 117 transmits through the collimator optical system 113 to be a collimated light, which is guided to the multiplexing optical system 107.

The dichroic mirror included in the multiplexing optical system 107 has an optical property to reflect a white illumination light and to transmit a narrowband light. The white illumination light and the narrowband light are mutually multiplexed via the multiplexing optical system 107 to be guided to the condensing optical system 109. The multiplexed white illumination light and narrowband light are coupled to one end of the light guide 201 of the image acquisition apparatus 20 by the condensing optical system 109.

The configuration of the light source apparatus 10 according to the present embodiment has been more specifically described above with reference to Fig. 8A to Fig. 10.

### [Degree of light amount distribution adjustment]

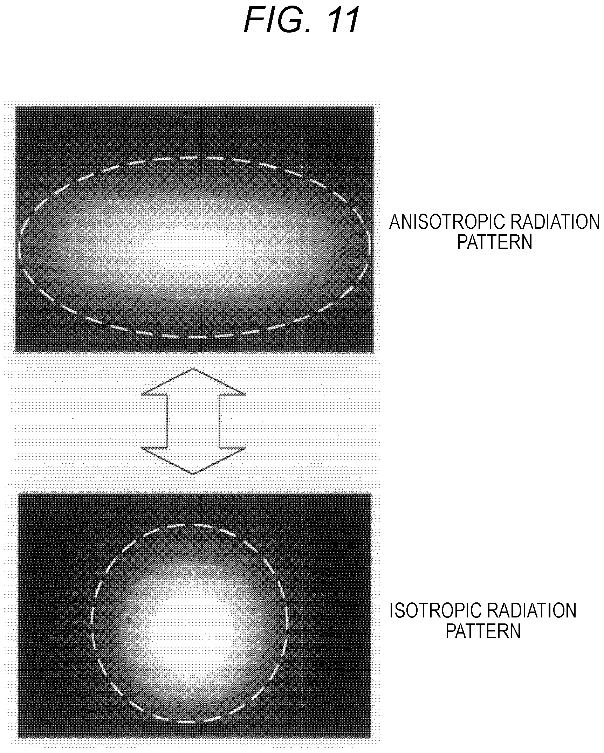

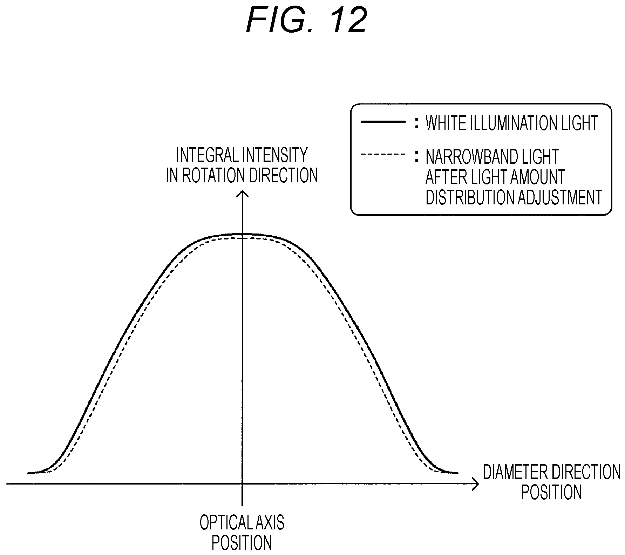

Fig. 11 is an expletory diagram for explaining radiation patterns of a narrowband light, and Fig. 12 is an explanatory diagram for explaining light amount distribution adjustment of a narrowband light according to the present embodiment.

A radiation pattern of a narrowband light turns into not the conventional isotropic radiation pattern as illustrated in the lower part of Fig. 11 but the anisotropic radiation pattern as illustrated in the upper part of Fig. 11 via each optical device as described above.

Here, it is preferable that a light amount distribution of a narrowband light is adjusted such that a distribution of an integrated value of the light amount integrated in the rotation direction in the radial direction of the light guide 201 is substantially the same between a white illumination light and the narrowband light as schematically illustrated in Fig. 12 at the incident end of the light guide 201 when the center axis of the light guide 201 is assumed as an optical axis and the center of rotation (and the center axis of the condensing optical system 109 is assumed as an optical axis) in the light source apparatus 10 according to the present embodiment. As the distribution shape of the integrated value of the light amount integrated in the rotation direction in the radial direction of the light guide 201 is similar between the white illumination light and the narrowband light, loss in light energy of the narrowband light can be further reduced.

Here, how much the distribution shape of the integrated value of the light amount is similar is not particularly limited, and the degree of similarity may be set as needed depending on an application of a light emitted from the light source apparatus 10, for example. Further, the degree of similarly of the distribution shape of the integrated value of the light amount can be quantified in any well-known method such as quantification by residual square sum of an integrated value of the light amount of a white illumination light and an integrated value of the light amount of a narrowband light in each radial direction position, for example, and the value found in the method is compared with a predetermined threshold thereby to objectively determine the degree of similarly of the distribution shape of the integrated value of the light amount (in other words, whether or not the distribution shape can be regarded as substantially the same).

### [Incident position of light flux into condensing optical system]

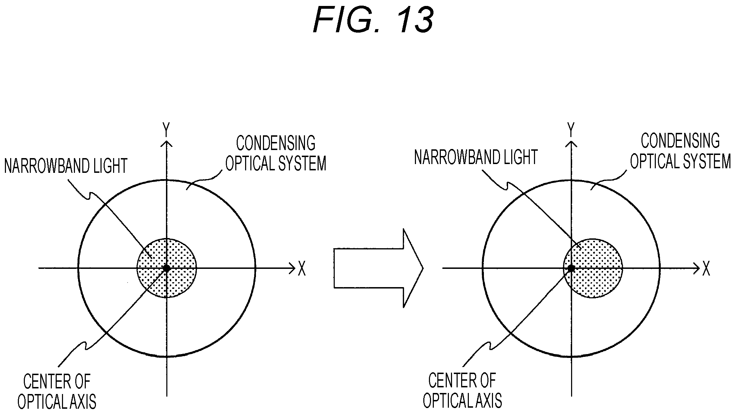

An incident position of a light flux into the condensing optical system will be described below with reference to Fig. 13 to Fig. 25B.

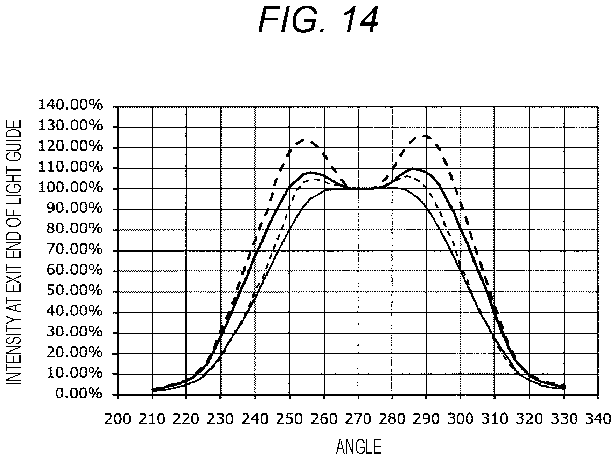

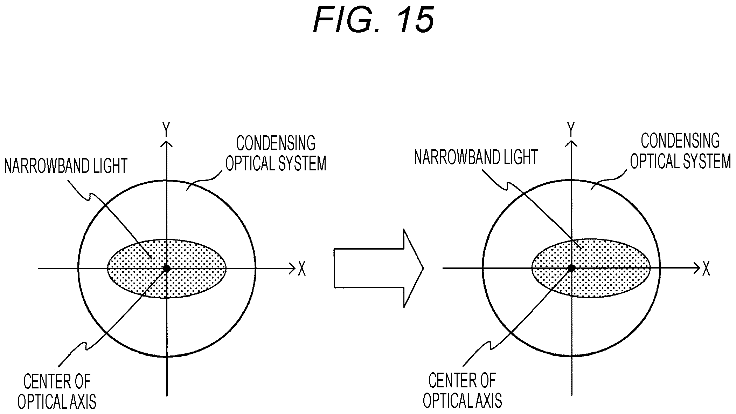

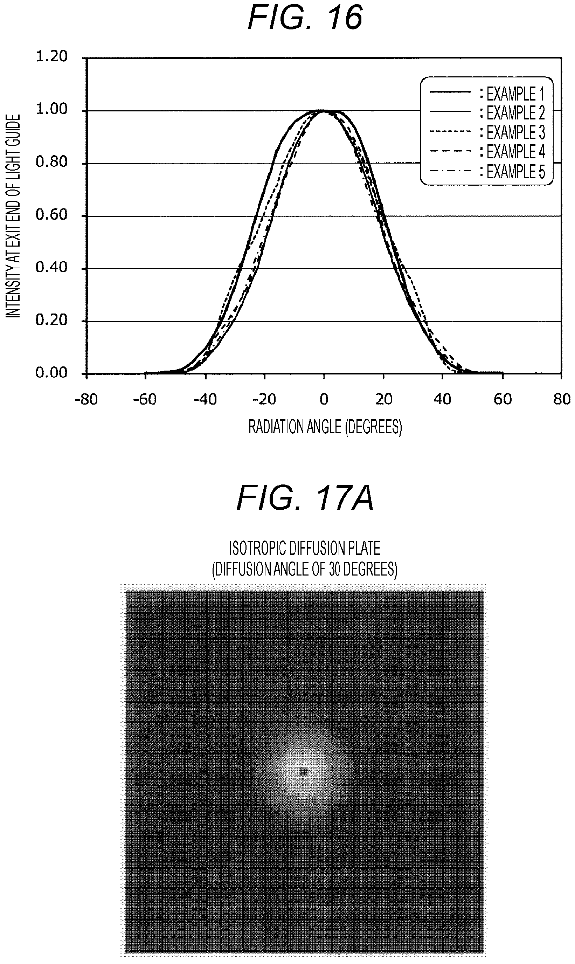

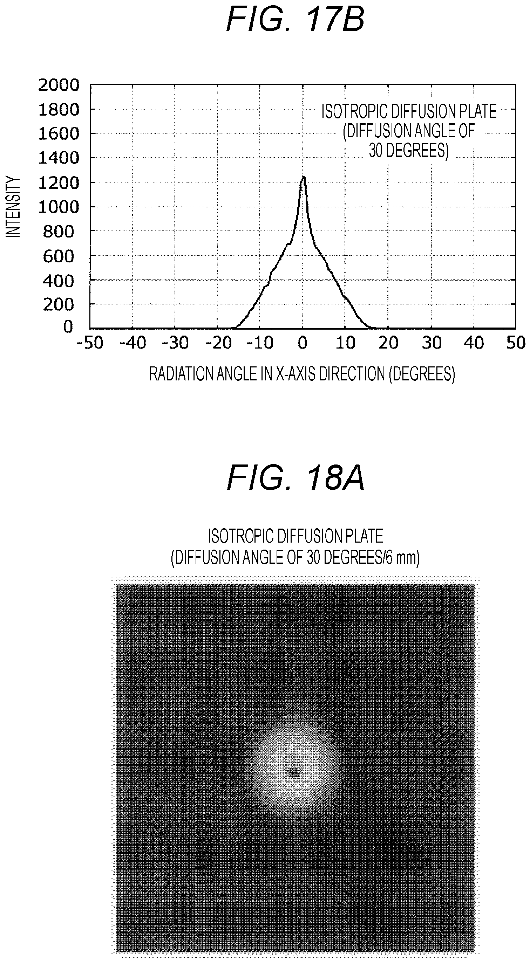

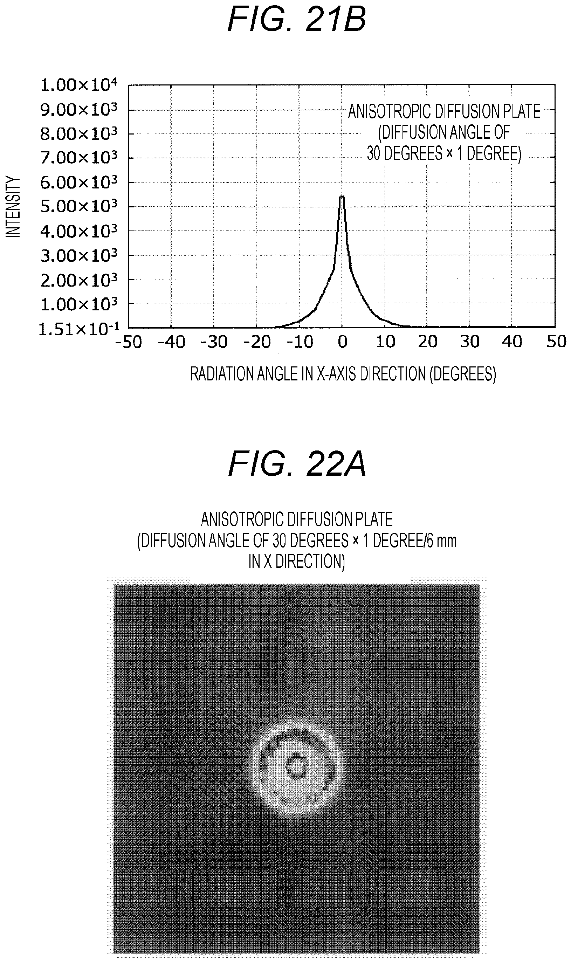

Fig. 13 is an explanatory diagram for explaining how a narrowband light is incident into the condensing optical system, and Fig. 14 is a graph illustrating exemplary intensity distributions of an isotropic narrowband light at the exit end of the light guide. Fig. 15 is an explanatory diagram for explaining how a narrowband light is incident into the condensing optical system according to the present embodiment, and Fig. 16 is a graph illustrating exemplary intensity distributions of a narrowband light at the exit end of the light guide. Fig. 17A, Fig. 18A, Fig. 19A, and Fig. 20A are explanatory diagrams illustrating exemplary intensity distributions of a narrowband light in a case where an isotropic diffusion plate is used, and Fig. 17B, Fig. 18B, Fig. 19B, and Fig. 20B are graphs illustrating exemplary intensity distributions of a narrowband light in a case where the isotropic diffusion plate is used. Fig. 21A, Fig. 21A, Fig. 22A, Fig. 23A, Fig. 24A, and Fig. 25A are explanatory diagrams illustrating exemplary intensity distributions of a narrowband light in a case where an anisotropic diffusion plate is used, and Fig. 21B, Fig. 22B, Fig. 23B, Fig. 24B, and Fig. 25B are graphs illustrating exemplary intensity distributions of a narrowband light in a case where the anisotropic diffusion plate is used.

The present inventors have additionally found that a laser light irradiation position is eccentric relative to the center of the optical axis of the condensing optical system thereby to change the radiation angle of the laser light transmitting through the condensing optical system. Here, the laser light is an isotropic Gaussian light, and thus the intensity of the laser light is the strongest at the center of the optical axis. Therefore, as schematically illustrated in Fig. 13, in a case where an incident position of an isotropic laser light (narrowband light) into the condensing optical system is offset in a direction (in the positive X-axis direction in Fig. 13, for example), the maximum point of the light intensity is offset from the center axis of the light guide. Consequently, the maximum point of the light intensity is offset from the center of the optical axis toward the periphery by the predetermined amount, and the center turns dark as illustrated in Fig. 14.

On the other hand, there will be considered a case in which an incident position of an anisotropic light flux of interest in the present embodiment into the condensing optical system is offset in a direction (in the positive X-axis direction in Fig. 15, for example) as schematically illustrated in Fig. 15. In this case, there is obtained an intensity distribution as illustrated in Fig. 16 in which when an intensity distribution in the Y-axis direction of the incident light flux is measured, the intensity is the strongest at the position of Y = 0 (or on the optical axis) . On the other hand, since the maximum intensity is not present at the position of X = 0 in the intensity distribution in the X-axis direction, when an intensity distribution in the X-axis direction is measured at the exit end of the light guide, a radiation distribution with the intensity at the center lowered is obtained as illustrated in Fig. 14. Finally, the intensity in the circumferential direction (rotation direction) is combined while the intensity in the radial direction is kept in the light guide, thereby realizing the radiation angle distribution with a higher degree of freedom and less loss of light. It is preferable that the multiplexed white illumination light and narrowband light are incident into a position offset from the center of the optical axis of the condensing optical system 109 when entering the condensing optical system 109 in the light source apparatus 10 according to the present embodiment because of the above reasons.

How an intensity distribution of a light flux at the incident end of the light guide 201 changes in a case where an incident position of the anisotropic light flux into the condensing optical system is eccentric will be calculated below by use of well-known light beam tracking simulation by way of an anisotropic diffusion plate used for the light amount distribution adjustment device 105. Additionally, the calculation conditions are set as follows. That is, a laser light is emitted on the anisotropic diffusion plate, and a diffused light from the anisotropic diffusion plate is made into a collimated light by the spherical lens with a focal length of 30 mm, and then is incident into the columnar glass rod having a lens with a numerical aperture NA = 0.7 and having a diameter of 4.5 mm × a length of 200 mm. Thereafter, an irradiation intensity on a screen of a radiated light from the exit end of the glass rod is calculated assuming the screen 30 mm away from the exit end of the glass rod. Additionally, for a diffusion angle of the anisotropic diffusion plate, the diffusion angle in the X-axis direction in Fig. 15 is assumed at 30 degrees and the diffusion angle in the Y-axis direction in Fig. 15 is assumed at 1 degree. Further, the shift amounts from the center of the optical axis are all assumed at 6 mm in the following examinations.

Further, examinations are similarly made for comparison also in a case where not an anisotropic diffusion plate but an isotropic diffusion plate with a diffusion angle of 1 degree and an isotropic diffusion plate with a diffusion angle of 30 degrees are used.

Examination results in a case where an isotropic diffusion plate is used will be first described with reference to Fig. 17A to Fig. 20B.

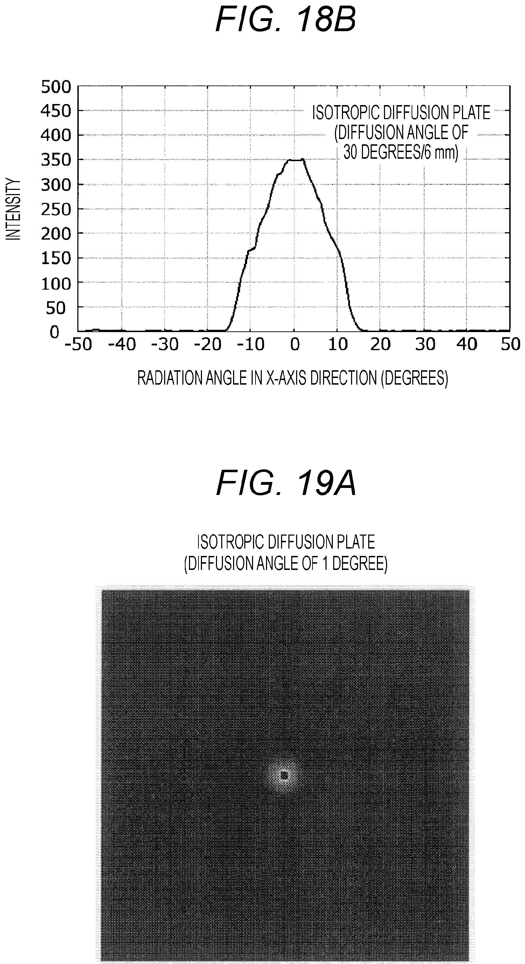

Fig. 17A and Fig. 17B illustrate simulation results in a case where a laser light is incident into the center of the optical axis of the condensing optical system by use of an isotropic diffusion plate with a diffusion angle of 30 degrees, and Fig. 18A and Fig. 18B illustrate simulation results in a case where a laser light is incident into a position 6 mm away from the center of the optical axis of the condensing optical system in the positive X-axis direction by use of an isotropic diffusion plate with a diffusion angle of 30 degrees . Further, Fig. 17A and Fig. 18A illustrate the intensity distributions on the screen, and Fig. 17B and Fig. 18B illustrate the intensity distributions in the X-axis direction on a plane passing through the center of the optical axis. As is clear by comparison between Figs. 17A and 17B and Figs. 18A and 18B, it can be confirmed that the radiation angle is larger when the incident position is laterally offset.

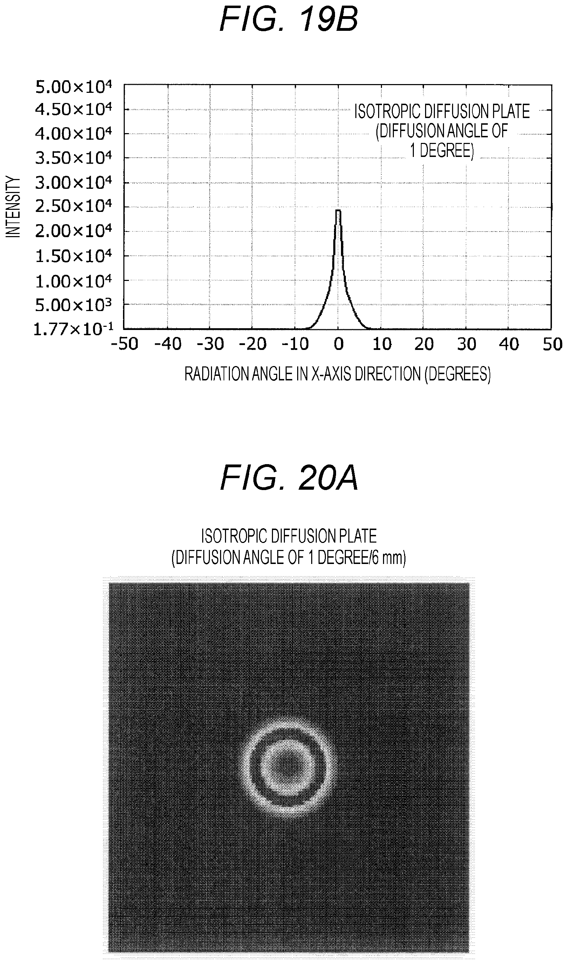

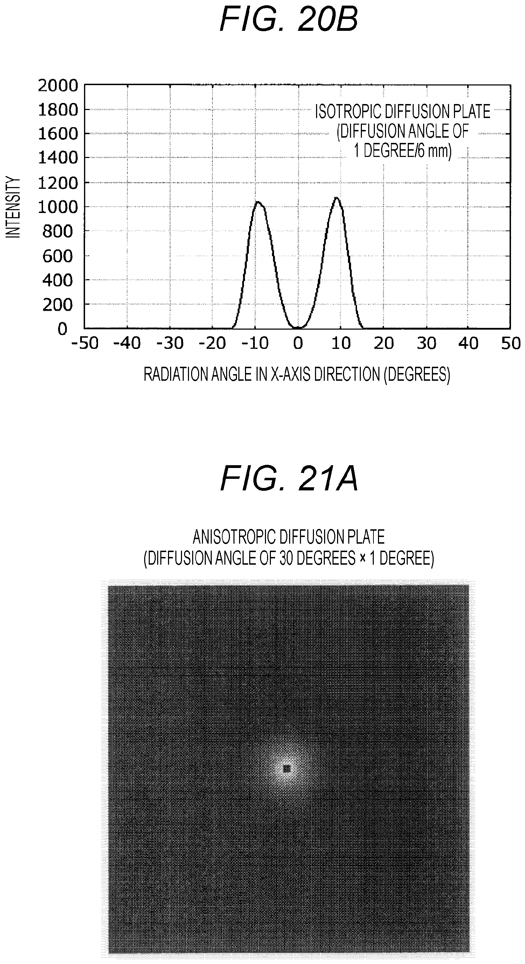

Further, Fig. 19A and Fig. 19B illustrate simulation results in a case where a laser light is incident into the center of the optical axis of the condensing optical system by use of an isotropic diffusion plate with a diffusion angle of 1 degree, and Fig. 20A and Fig. 20B illustrate simulation results in a case where a laser light is incident into a position 6 mm away from the center of the optical axis of the condensing optical system in the positive X-axis direction by use of an isotropic diffusion plate with a diffusion angle of 1 degree. As is clear by comparison between Figs. 19A and 19B and Figs. 20A and 20B, it can be seen that the peak position of the intensity changes from the position at the center of the optical axis (radiation angle of 0 degrees) to the position at ±10 degrees therefrom when the incident position is laterally offset.

In the examples of Fig. 17A to Fig. 20B, an isotropic diffusion plate is used so that a change in radiation angle is also isotropic as illustrated in each Figure and a similar change in radiation angle is indicated also in the Y-axis direction, for example. Thus, in a case where such a light flux is incident into the light guide, the maximum point of the light intensity is offset from the center of the optical axis toward the periphery by the predetermined amount and the center turns dark as described earlier.

Fig. 21A to Fig. 25B illustrate simulation results in a case where an anisotropic diffusion plate is used. Additionally, it should be noted that a laser light as an oval light flux by the anisotropic diffusion plate is incident into the columnar glass rod and thus the intensity distribution on the screen reflects the shape of the glass rod to be circular in the present simulations.

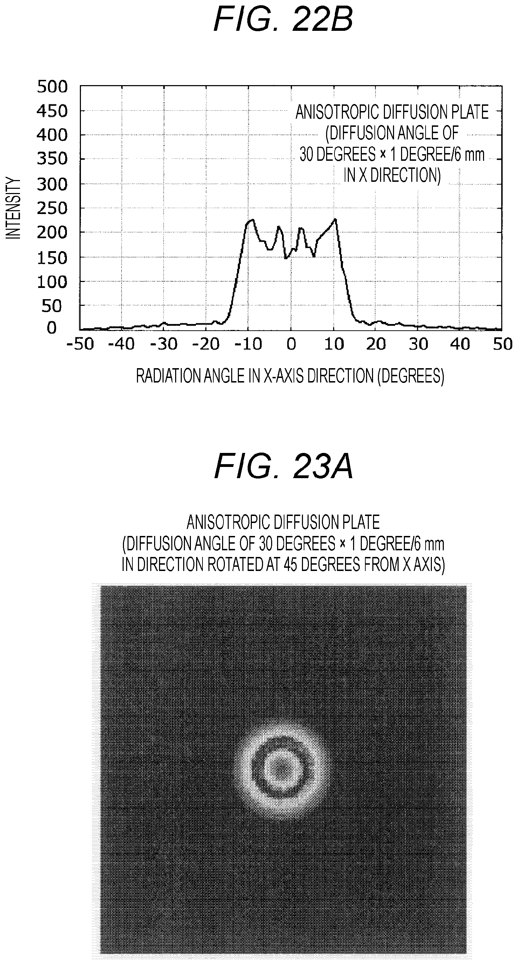

Fig. 21A and Fig. 21B illustrate simulation results in a case where a laser light is incident into the center of the optical axis of the condensing optical system by use of an anisotropic diffusion plate with a diffusion angle of 30 degrees in the X-axis direction × a diffusion angle of 1 degree in the Y-axis direction, and Fig. 22A and Fig. 22B illustrate simulation results in a case where a laser light is incident into a position 6 mm away from the center of the optical axis of the condensing optical system in the X-axis direction. As is clear by comparison between Figs. 21A and 21B and Figs. 22A and 22B, it can be confirmed that an incident position is laterally offset so that the radiation angle is larger.

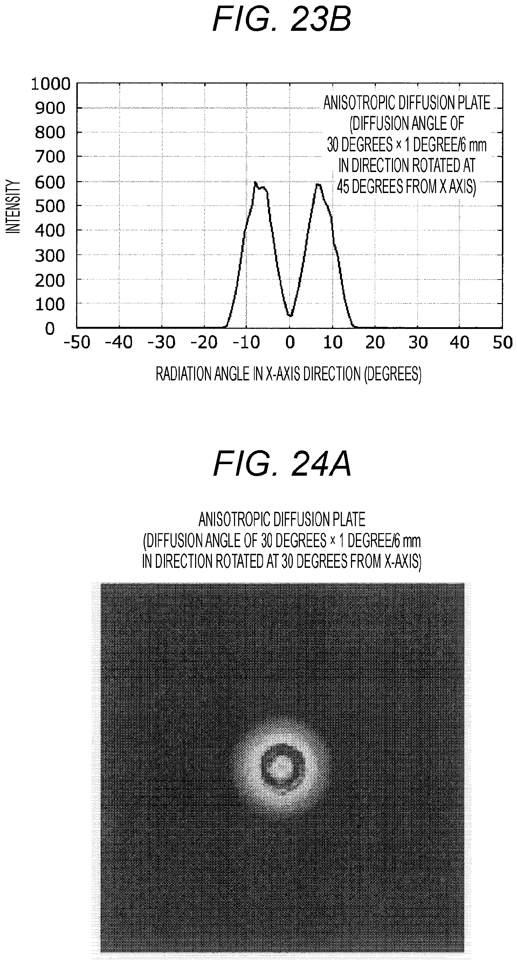

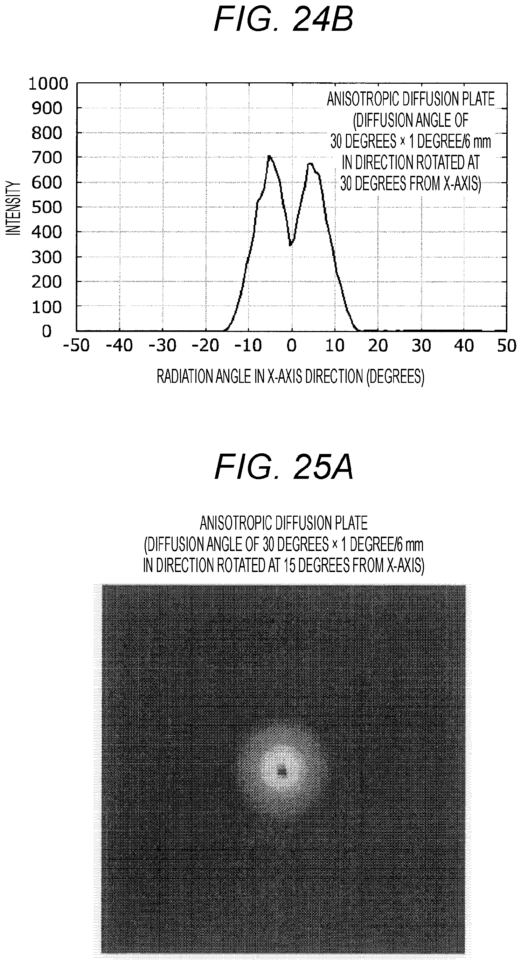

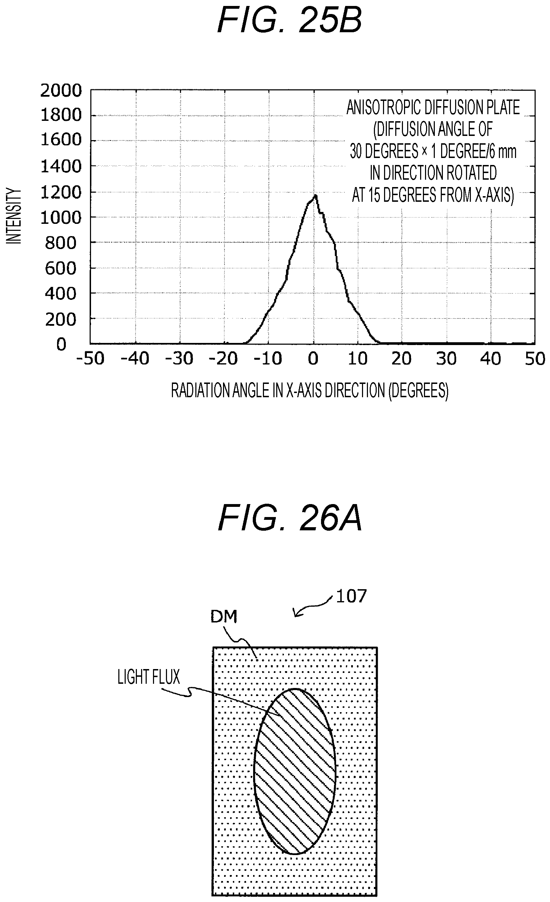

Further, Fig. 23A and Fig. 23B illustrate simulation results in a case where a laser light is incident into a position 6 mm offset from the optical center in a direction rotated by 45 degrees from the X-axis in the condensing optical system, Fig. 24A and Fig. 24B illustrate simulation results in a case where a laser light is incident into a position 6 mm offset from the optical center in a direction rotated by 30 degrees from the X-axis, and Fig. 25A and Fig. 25B illustrate simulation results in a case where a laser light is incident into a position 6 mm offset from the optical center in a direction rotated by 15 degrees from the X-axis. As is clear from the simulation results, it can be seen that the incident position of the laser light is changed in the condensing optical system thereby to adjust the radiation pattern of the laser light to a desired shape.

Further, in the actual light source apparatus 10, a narrowband light transmitting through the light amount distribution adjustment device 105 is different in terms of a radiation pattern in the X-axis direction and a radiation pattern in the Y-axis direction, and the narrowband light is coupled to the light guide 201 of the image acquisition apparatus 20 while the anisotropy is kept. As described earlier, the intensity in the circumferential direction is combined in the light guide 201 while the intensity in the radial direction of the light guide is kept. Consequently, a radiation angle distribution with a high degree of freedom and less loss of light energy can be realized.

### [Multiplexing optical system 107]

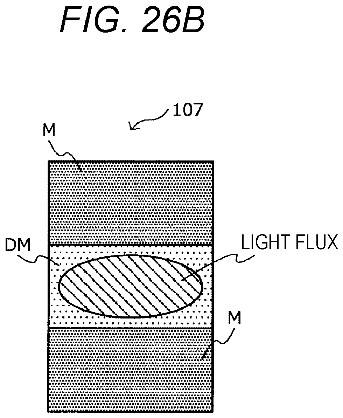

Subsequently, the multiplexing optical system 107 (more specifically, the dichroic mirror) usable in the light source apparatus 10 according to the present embodiment will be briefly described with reference to Fig. 26A and Fig. 26B. Fig. 26A is an explanatory diagram schematically illustrating an exemplary multiplexing optical system in the light source apparatus according to the present embodiment, and Fig. 26B is an explanatory diagram schematically illustrating other exemplary multiplexing optical system in the light source apparatus according to the present embodiment.

There will be considered a case in which the dichroic mirror provided as the multiplexing optical system 107 is arranged at an angle of 45 degrees relative to the optical axis as illustrated in Fig. 8C, Fig. 9B, and Fig. 10. In this case, as schematically illustrated in Fig. 26A, an optical device in which an entire light incident plane functions as a dichroic mirror DM is generally used in many cases. In a case where a substantially-circular light flux is incident into the incident plane of the optical device, if the dichroic mirror DM is arranged at an angle of 45 degrees relative to the optical axis, the shape of the incident light flux is assumed to be oval as illustrated in Fig. 26A.

On the other hand, the radiation profile (intensity distribution) of a narrowband light is anisotropic in the light source apparatus 10 according to the present embodiment as described earlier, and thus the irradiation region of the narrowband light is smaller than the irradiation region of a white illumination light in the dichroic mirror DM provided as the multiplexing optical system 107.

The intensity distribution of a narrowband light is anisotropic in the light source apparatus 10 according to the present embodiment as described above, and thus the irradiation region of the narrowband light is smaller than the irradiation region of a white illumination light in the dichroic mirror provided as the multiplexing optical system 107.

In a case where the dichroic mirror DM is arranged at an angle of 45 degrees relative to the optical axis, the circular irradiation region onto the plane of the dichroic mirror DM is actually 2^{0.5} longer in the long-side direction, but projection of a narrowband light or a white illumination light onto the optical axis will be assumed for simplicity. At this time, there will be considered a case in which a light flux as illustrated in the upper part of Fig. 11 is incident into the dichroic mirror DM, for example. At this time, the size of the dichroic mirror required for transmitting a narrowband light and reflecting a white illumination light is as large as the irradiation region of a narrowband light as illustrated in Fig. 26B, and a narrowband light is not incident into other region and thus the dichroic mirror DM does not have to be employed. Thus, a complex mirror as illustrated in Fig. 26B, which is used as the dichroic mirror DM only for the irradiation region of a narrowband light and as the total reflection mirror M for totally reflecting a white illumination light for other part, may be used in the light source apparatus 10 according to the present embodiment.

An optical system including the complex mirror as illustrated in Fig. 26B is used as the multiplexing optical system 107, thereby achieving a further increase in the light amount of a white illumination light guided to the condensing optical system 109 and reducing cost due to the downsized dichroic mirror DM.

The multiplexing optical system 107 usable in the light source apparatus 10 according to the present embodiment has been briefly described above.

### (Conclusion)

As described above, a light amount distribution adjustment device for giving anisotropy to a radiation angle of distribution of a narrowband light, such as a group of lenses including at least two cylindrical lenses, an anisotropic diffusion plate, or a multi-lens array in a cylindrical lens shape, is used in the light source apparatus 10 according to the embodiment of the present disclosure, thereby realizing a uniform irradiation profile between a white illumination light and a narrowband light and efficiently using the lights while restricting an increase in manufacture cost.

The preferred embodiment of the present disclosure has been described above in detail with reference to the accompanying drawings, but the technical scope of the present disclosure is not limited to the example. It is clear that those skilled in the art according to the present disclosure can assume various changes and modifications within the scope of the technical spirit described in CLAIMS and these of course belong to the technical scope of the present disclosure.

Further, the effects described in the present specification are merely explanatory or exemplary, and are not restrictive. That is, the technology according to the present disclosure can provide other effects clear to those skilled in the art from the description of the present specification together with the above effects or instead of the above effects.

Additionally, the following configurations also belong to the technical scope of the present disclosure.
(1) A light source apparatus including:
   a white illumination light source configured to emit a white illumination light;
   one or more narrowband light sources configured to emit a narrowband light with a narrower wavelength band than the white illumination light;
   a multiplexing optical system configured to multiplex the white illumination light and the narrowband light;
   a condensing optical system configured to couple the multiplexed white illumination light and narrowband light to a light guide provided in an image acquisition apparatus and configured to guide an illumination light into the image acquisition apparatus; and
   a light amount distribution adjustment device provided on an optical axis between the narrowband light source and the multiplexing optical system and configured to adjust a light amount distribution of the narrowband light,
   in which the light guide is a bundle-type light guide in which multimode optical fibers are bundled or a liquid light guide filling a liquid therein, and
   the light amount distribution adjustment device adjusts a light amount distribution of the narrowband light such that a radiation angle distribution of the narrowband light transmitting through the light amount distribution adjustment device is anisotropic.
(2) The light source apparatus according to (1), in which when a center axis of the light guide is assumed as an optical axis and the center of rotation, the light amount distribution adjustment device adjusts a light amount distribution of the narrowband light such that a radiation angle distribution of the narrowband light transmitting through the light amount distribution adjustment device is anisotropic and a distribution of an integrated value of the light amount integrated in a rotation direction in a radial direction of the light guide is substantially the same between the white illumination light and the narrowband light at an incident end of the light guide.
(3) The light source apparatus according to (1) or (2), in which the multiplexed white illumination light and narrowband light are incident into a position offset from the center of an optical axis of the condensing optical system when entering the condensing optical system.
(4) The light source apparatus according to any one of (1) to (3), in which the light amount distribution adjustment device is at least any of a group of lenses including at least two cylindrical lenses, an anisotropic diffusion plate with an anisotropic diffusion property, and a multi-lens array in a cylindrical lens shape.
(5) The light source apparatus according to any one of (1) to (4), in which collimator optical systems for turning the narrowband light and the white illumination light into collimated lights are provided on an optical axis between the light amount distribution adjustment device and the multiplexing optical system and on an optical axis between the white illumination light source and the multiplexing optical system, respectively.
(6) The light source apparatus according to any one of (1) to (5), in which the multiplexing optical system includes a complex mirror provided with a dichroic mirror configured to transmit the narrowband light and to reflect the white illumination light in a part where the narrowband light with a light amount distribution adjusted is emitted, and provided with a total reflection mirror configured to reflect the white illumination light other than in the part where the dichroic mirror is provided.
(7) The light source apparatus according to any one of (1) to (6), in which the white illumination light source is a fluorescent light source in which a predetermined fluorescent substance is combined with a predetermined light source, and
   the narrowband light source is a solid-state light source.
(8) The light source apparatus according to any one of (1) to (7), in which the image acquisition apparatus is an endoscope or a microscope.
(9) A light amount distribution adjustment method including:
   putting a narrowband light emitted from one or more narrowband light sources configured to emit a narrowband light with a narrower wavelength band than a white illumination light emitted from a white illumination light source into a light amount distribution adjustment device configured to adjust a light amount distribution of the narrowband light;
   multiplexing the narrowband light with the adjusted light amount distribution and the white illumination light by a multiplexing optical system; and
   coupling the multiplexed white illumination light and narrowband light to a light guide by a condensing optical system configured to couple the lights to the light guide provided in an image acquisition apparatus and configured to guide an illumination light into the image acquisition apparatus,
   in which the light guide is a bundle-type light guide in which multimode optical fibers are bundled or a liquid light guide filling a liquid therein, and
   the light amount distribution adjustment device adjusts a light amount distribution of the narrowband light such that a radiation angle distribution of the narrowband light transmitting through the light amount distribution adjustment device is anisotropic.
(10) An image acquisition system including:
   a light source apparatus configured to multiplex a white illumination light and a narrowband light with a narrower wavelength band than the white illumination light and to emit the multiplexed lights to the outside; and
   an image acquisition apparatus configured to acquire an image of an object to be observed by use of a light emitted from the light source apparatus,
   in which the image acquisition apparatus includes a light guide configured to guide an illumination light into the image acquisition apparatus,
   the light guide is a bundle-type light guide in which multimode optical fibers are bundled or a liquid light guide filling a liquid therein,
   the light source apparatus includes:
      a white illumination light source configured to emit the white illumination light;
      one or more narrowband light sources configured to emit the narrowband light;
      a multiplexing optical system configured to multiplex the white illumination light and the narrowband light;
      a condensing optical system configured to couple the multiplexed white illumination light and narrowband light to the light guide provided in the image acquisition apparatus; and
      a light amount distribution adjustment device provided on an optical axis between the narrowband light source and the multiplexing optical system and configured to adjust a light amount distribution of the narrowband light, and
      the light amount distribution adjustment device adjusts a light amount distribution of the narrowband light such that a radiation angle distribution of the narrowband light transmitting through the light amount distribution adjustment device is anisotropic.

### REFERENCE SIGNS LIST

- 1: Image acquisition system
- 10: Light source apparatus
- 20: Image acquisition apparatus
- 101: White illumination light source
- 103: Narrowband light source
- 105: Light amount distribution adjustment device
- 107: Multiplexing optical system
- 109: Condensing optical system
- 111: Cylindrical lens
- 113: Collimator optical system
- 115: Anisotropic diffusion plate
- 117: Multi-lens array
- 201: Light guide
- 203: Image acquisition unit

## Claims

1. A light source apparatus comprising:
a white illumination light source configured to emit a white illumination light;
one or more narrowband light sources configured to emit a narrowband light with a narrower wavelength band than the white illumination light;
a multiplexing optical system configured to multiplex the white illumination light and the narrowband light;
a condensing optical system configured to couple the multiplexed white illumination light and narrowband light to a light guide provided in an image acquisition apparatus and configured to guide an illumination light into the image acquisition apparatus; and
a light amount distribution adjustment device provided on an optical axis between the narrowband light source and the multiplexing optical system and configured to adjust a light amount distribution of the narrowband light,
wherein the light guide comprises a bundle-type light guide in which multimode optical fibers are bundled or a liquid light guide filling a liquid therein, and
the light amount distribution adjustment device adjusts a light amount distribution of the narrowband light such that a radiation angle distribution of the narrowband light transmitting through the light amount distribution adjustment device is anisotropic.

2. The light source apparatus according to claim 1,
wherein when a center axis of the light guide is assumed as an optical axis and the center of rotation, the light amount distribution adjustment device adjusts a light amount distribution of the narrowband light such that a radiation angle distribution of the narrowband light transmitting through the light amount distribution adjustment device is anisotropic and a distribution of an integrated value of the light amount integrated in a rotation direction in a radial direction of the light guide is substantially the same between the white illumination light and the narrowband light at an incident end of the light guide.

3. The light source apparatus according to claim 1,
wherein the multiplexed white illumination light and narrowband light are incident into a position offset from the center of an optical axis of the condensing optical system when entering the condensing optical system.

4. The light source apparatus according to claim 1,
wherein the light amount distribution adjustment device comprises at least any of a group of lenses including at least two cylindrical lenses, an anisotropic diffusion plate with an anisotropic diffusion property, and a multi-lens array in a cylindrical lens shape.

5. The light source apparatus according to claim 1,
wherein collimator optical systems for turning the narrowband light and the white illumination light into collimated lights are provided on an optical axis between the light amount distribution adjustment device and the multiplexing optical system and on an optical axis between the white illumination light source and the multiplexing optical system, respectively.

6. The light source apparatus according to claim 1,
wherein the multiplexing optical system includes a complex mirror provided with a dichroic mirror configured to transmit the narrowband light and to reflect the white illumination light in a part where the narrowband light with a light amount distribution adjusted is emitted, and provided with a total reflection mirror configured to reflect the white illumination light other than in the part where the dichroic mirror is provided.

7. The light source apparatus according to claim 1,
wherein the white illumination light source comprises a fluorescent light source in which a predetermined fluorescent substance is combined with a predetermined light source, and
the narrowband light source comprises a solid-state light source.

8. The light source apparatus according to claim 1,
wherein the image acquisition apparatus comprises an endoscope or a microscope.

9. A light amount distribution adjustment method comprising:
putting a narrowband light emitted from one or more narrowband light sources configured to emit a narrowband light with a narrower wavelength band than a white illumination light emitted from a white illumination light source into a light amount distribution adjustment device configured to adjust a light amount distribution of the narrowband light;
multiplexing the narrowband light with the adjusted light amount distribution and the white illumination light by a multiplexing optical system; and
coupling the multiplexed white illumination light and narrowband light to a light guide by a condensing optical system configured to couple the lights to the light guide provided in an image acquisition apparatus and configured to guide an illumination light into the image acquisition apparatus,
wherein the light guide comprises a bundle-type light guide in which multimode optical fibers are bundled or a liquid light guide filling a liquid therein, and
the light amount distribution adjustment device adjusts a light amount distribution of the narrowband light such that a radiation angle distribution of the narrowband light transmitting through the light amount distribution adjustment device is anisotropic.

10. An image acquisition system comprising:
a light source apparatus configured to multiplex a white illumination light and a narrowband light with a narrower wavelength band than the white illumination light and to emit the multiplexed lights to the outside; and
an image acquisition apparatus configured to acquire an image of an object to be observed by use of a light emitted from the light source apparatus,
wherein the image acquisition apparatus includes a light guide configured to guide an illumination light into the image acquisition apparatus,
the light guide comprises a bundle-type light guide in which multimode optical fibers are bundled or a liquid light guide filling a liquid therein,
the light source apparatus includes:
a white illumination light source configured to emit the white illumination light;
one or more narrowband light sources configured to emit the narrowband light;
a multiplexing optical system configured to multiplex the white illumination light and the narrowband light;
a condensing optical system configured to couple the multiplexed white illumination light and narrowband light to the light guide provided in the image acquisition apparatus; and
a light amount distribution adjustment device provided on an optical axis between the narrowband light source and the multiplexing optical system and configured to adjust a light amount distribution of the narrowband light, and
the light amount distribution adjustment device adjusts a light amount distribution of the narrowband light such that a radiation angle distribution of the narrowband light transmitting through the light amount distribution adjustment device is anisotropic.
